# EUROPÄISCHE PATENTSCHRIFT

(11) **EP 1 513 563 B1**
(45) Veröffentlichungstag und Bekanntmachung des Hinweises auf die Patenterteilung: **19.10.2005**
(21) Anmeldenummer: 04732956.0
(22) Anmeldetag: 14.05.2004
(51) Int. Cl.: A61L 27/00, A61L 27/30, A61L 27/34, A61L 29/08, A61L 29/10

(54) **IMPLANTAT MIT ANTIBIOTISCHER LANGZEITWIRKUNG**
IMPLANT HAVING A LONG-TERM ANTIBIOTIC EFFECT
IMPLANT A ACTION LONGUE DUREE ANTIBIOTIQUE

(30) Priorität: 15.05.2003 DE 10323676
(43) Veröffentlichungstag der Anmeldung: 16.03.2005
(73) Patentinhaber: Aesculap AG & Co. KG, 78532 Tuttlingen (DE)
(72) Erfinder: GOLDMANN, Helmut, 78532 Tuttlingen (DE)
(74) Vertreter: Patentanwälte Ruff, Wilhelm, Beier, Dauster & Partner
(86) Internationale Anmeldenummer: PCT/EP2004/005162
(87) Internationale Veröffentlichungsnummer: WO 2004/101010

(56) Entgegenhaltungen:
- DE-A- 3 228 849
- US-A1- 2003 049 300
- US-A1- 2003 050 689
- US-B1- 6 296 863
- US-B1- 6 530 951

## Beschreibung

Die Erfindung betrifft ein Implantat mit antibiotischer Langzeitwirkung. Die Infektion bei der Implantierung von Prothesen und anderen lmplantaten stellt einen Risikofaktor dar, der sowohl von Ärzten als auch von Patienten gefürchtet ist. Die Häufigkeit einer Implantatinfektion beträgt etwa 0,5 bis 5 %. Risikofaktoren sind bei künstlichen Gefäßimplantaten z.B. Notfalloperationen, eine subkutane Prothesenposition oder etwa eine Positionierung der Prothese in der Leistengegend. Man unterscheidet zwischen Frühinfektion, die in der Regel während einer Zeitdauer bis zu 4 Monaten nach der Implantation auftreten und sogenannten Spätinfektionen, die sich in größeren Zeiträumen nach der Implantation bemerkbar machen. Klinische Berichte belegen z.B. für Infektionen der Aorta einen Auftritt nach 25 - 70 Monaten. In aorto-femoraler Position beträgt die mittlere Zeit bis zum Ausbruch der Infektion 41 Monate. Extra kavitäre Protheseninfekte treten früher auf (innerhalb 7 Monate). Zu den Keimen, die solche Infektionen verursachen gehören insbesondere Staphylococcus aureus, Staphylococcus epidermidis und Escherichia coli. Die Infizierung erfolgt in der Regel durch intraoperative Kontamination. Sie kann aber auch postoperativ erfolgen, insbesondere bei einer nicht völlig ausgeheilten Infektion des Patienten. Die Keime bzw. Mikroorganismen neigen dazu, sich an die Prothesenoberfläche anzuheften. Sie können dabei eine Mikrokolonnie innerhalb eines Biofilmes bilden, wobei sie nach außen über längere Zeit abgeschlossen sein können. Insbesondere dann, wenn der Patient aus anderen Gründen geschwächt ist, kann es zur virulenten Infektion und zu Entzündungsreaktionen unter Einschluss der Perigraft-Gewebes und der Anastomosenregion kommen.

Es ist bekannt, dass Silber eine antibiotische Wirkung besitzt. Silbersalze und metallisches Silber werden deshalb vielfach bei der Bekämpfung von Mikroorganismen eingesetzt. So ist es beispielsweise aus der WO 93/07924 bekannt, Gegenstände aus Kunststoffen, Metall und Keramik, die in den Körper gelangen, wie Fixiereinrichtungen, Nägel und Stifte, Katheter, Stents, Tracheostomierohre, Shunts, perkutane Verbinder, Wunddrainageeinrichtungen, Dentalimplantate und dergleichen mit einer bakteriziden Komponente, insbesondere aus Platin, Iridium, Gold, Silber, Quecksilber, Kupfer, Jod sowie deren Legierungen, Verbindungen und Oxide zu versehen. Die entsprechenden Stoffe werden in Form von ionisierten Atomen in einer Vakuumkammer durch ion-beam-assisted deposition (IBAD) aufgetragen. Biomedizinische Implantate mit ähnlichen Bakterizidenoberflächen sind in der US 5,492,763 beschrieben. Dort werden als biomedizinische Gegenstände unter anderem metallische Nadeln, urologische Katheter, perkutane Klammern sowie keramische und metallische Gegenflächen von Hüft- und Kniegelenken genannt.

Ferner ist es aus der WO 81/02667 bekannt, Implantate, wie beispielsweise künstliche Gelenke mit einer Oberflächenbeschichtung aus Silber oder Silberlegierungen mit einer Schichtdicke von 25 bis 500 Å zu versehen, um einerseits ein Bakterienwachstum zu verhindern, andererseits zu vermeiden, dass die Silbermenge so groß ist, dass umliegendes Bindegewebe beschädigt wird.

US 2003/0049300 beschreibet medizinische Vorrichtungen, die mit einer Polymerschicht verschen sind, die Silber enthält.

Aus der US 5,464,438 ist es weiterhin bekannt, Implantate aus textilem Material mit metallischem Gold zu bedampfen, um die Thrombosegefahr zu verringern.

Normalerweise werden textile Implantate, insbesondere dann, wenn sie als Ersatz für Hohlorgane, insbesondere Leitungsbahnen dienen, wozu hauptsächlich Gefäßprothesen gehören, mit abdichtenden Beschichtungen versehen, um zumindest anfänglich die Poren der textilen Prothesen zu verschließen. Es wurde vorgeschlagen, bakterizide Stoffe in das Beschichtungsmaterial einzulagern, um auf diese Weise Infektionen nach der Implantation verhindern zu können. Solche Beschichtungen, die unter anderem auch Silberionen enthalten können, ergeben sich aus der WO 00/32247.

Der Erfindung liegt die Aufgabe zugrunde, ein Implantat, insbesondere eine Gefäßprothese, mit antibiotischer Langzeitwirkung zu schaffen, wobei das Implantat in üblicherweise gehandhabt werden soll und die Infektionsgefahr auf ein Minimum reduziert ist.

Gegenstand der Erfindung ist ein Implantat mit antibiotischer bzw. antimikrobieller Langzeitwirkung, insbesondere eine Gefäßprothese, mit einer die Form des Implantats vorgebenden Grundstruktur aus im wesentlichem nicht oder nur langsam resorbierbarem polymerem Material und einer Beschichtung aus einem resorbierbarem Material, wobei sich auf dem polymeren Material und unter der Beschichtung eine Schicht aus metallischem Silber befindet.

An sich war zu befürchten, dass eine Interaktion zwischen der resorbierbaren Beschichtung und der Silberschicht eintritt. Dies ist auch tatsächlich der Fall, insbesondere dann, wenn die resorbierbare Schicht aus biologischem Material, wie Gelatine und Collagen besteht. Es wurde jedoch gefunden, dass diese Interaktion eher von Vorteil ist. So haben, wie später noch erläutert wird, Vergleichsversuche gezeigt, dass die Abgabe von Silberionen bei mit einer resorbierbaren Schicht versehenen Prothesen im Vergleich zu nur mit einer Silberschicht versehenen Prothesen anfänglich sehr hoch ist, selbst dann, wenn in der resorbierbaren Schicht kein Silber eingelagert war. Offenbar findet eine Korrosion der Silberschicht durch die Bestandteile der resorbierbaren Schicht statt, was während der Lagerung der Prothese bis ihrer Verwendung erfolgen kann. Dabei lagern sich freigesetzte Silberionen in der resorbierbaren Schicht ab und werden mit deren Abbau beschleunigt freigegeben. Wird die Silberschicht ausreichend stark bemessen, dann beeinträchtigt dies die Langzeitwirkung der Silberschicht nicht, so dass die bakterizide Wirkung der Silberschicht auch dann noch über lange Zeit anhält, wenn die resorbierbare Schicht aufgelöst ist.

Die Silberschicht ist mit Vorteil auf der Oberfläche des Polymermaterials fest haftend und insbesondere in dieser verankert. Dies kann durch die aus dem Stand der Technik bekannten Bedampfungsverfahren, insbesondere durch das oben erwähnte IBAD-Verfahren bewerktstelligt werden. Deshalb ist die Silberschicht bevorzugt auf die Polymeroberfläche aufgedampft. Besonders bevorzugt ist es, wenn die Silberatome der Silberschicht in die Polymeroberfläche der Grundstruktur eingeprägt sind. Dies kann mit Vorteil durch Beschuss der Polymeroberfläche z.B. mit Argonionen während der Bedampfung bewerktstelligt werden.

Die Silberschicht deckt die Polymeroberfläche zumindest an den Stellen, an denen sie nach Implantation mit Bindegewebe in Kontakt kommt, vorzugsweise vollständig ab. Insbesondere liegt zumindest in diesen Bereichen geschlossene Silberschichten vor. Bei der bevorzugten Ausführungsform ist die Silberschicht so dick, dass sie in vivo, d.h. nach der Implantation, eine Verweildauer auf der Polymeroberfläche von mehr als einem Jahr, insbesondere mehr als 2 Jahren, besitzt und während dieser Zeit Silberionen abgibt. Mit besonderem Vorteil ist die Silberschicht so dick bemessen, dass beim Abbau im Körper nur ca. 5 bis 10 %, insbesondere 7 bis 8 % der Schichtdicke pro Jahr abgetragen werden. Es hat sich nämlich gezeigt, dass die in der Literatur beschriebene mögliche Schädigung des umliegenden Gewebes nicht eine Funktion der Schichtdicke der Silberschicht ist. Auch dickere Schichten geben pro Zeiteinheit nicht mehr Silberionen ab, dafür aber über eine längere Zeit. Bewährt haben sich Schichtdicken im Bereich von 1000 Å bis 2500 Å, insbesondere solche, die bei ca. 1300 Å liegen. Solche Schichtdicken zeigen eine gute Langzeitwirkung. Die Schichtdicke kann auch größer sein und bis zu 4000 Å und mehr betragen, doch bringen größere Schichtdicken keine wesentlichen zusätzlichen Vorteile. Geringere Schichtdicken können, insbesondere wegen der Wechselwirkung mit der resorbierbaren Schicht, zu einem unerwünscht frühen Nachlassen der Langzeitwirkung führen.

Als Polymermaterial für die Grundstruktur kommen die üblichen bei Implantaten, insbesondere Gefäßprothesen, verwendeten Polymere in Frage, wie Polyester, Polytetrafluorethylen, Polyurethan und in besonderen Fällen auch Polyamide, wobei in der Regel Polyester bevorzugt sind. Die Silberschicht befindet sich vorzugsweise zumindest auf der Seite bzw. den Seiten des Polymermaterials, die dem Bindegewebe zugewandt ist. Die Silberschicht besteht vorzugsweise aus reinem elementarem Silber.

Die Grundstruktur des Implantats ist insbesondere dann, wenn es sich um eine Gefäßprothese handelt, aber auch bei Herniennetzen, Patches u.dgl., porös, und die resorbierbare Schicht ist eine die Poren des Implantats abdichtende Imprägnierung. Wie oben bereits erwähnt, kann die resorbierbare Schicht aus biologischem Material gebildet sein, das gegebenenfalls auch vernetzt sein kann. Als Materialien kommen hier insbesondere Collagen, Gelatine und Albumin in Frage. Alternativ oder in Kombination kann die resorbierbare Schicht auch von in vivo abbaubaren oder resorbierbaren synthetischen Polymeren und Copolymeren gebildet sein. Hier kommen neben mindestens teilweise wasserlöslichen Polymeren, wie Polyvinylalkohol, Carboxymethylcellulose, vorwiegend die Polymere und Copolymere von Hydroxysäuren in Frage. Hier handelt es sich insbesondere um Polymere und Copolymere von Glykolid, Lactid, Epsilon-caprolacton, Trimethylcarbonat und Paradioxanon. Auch Mischungen der Polymere kommen in Frage. Durch geeignete Wahl der Polymere kann die gewünschte Resorptionsdauer eingestellt werden. Diese liegt vorzugsweise innerhalb von 4 Monaten und insbesondere innerhalb von 40 Tagen. Eine solche Zeit ist günstig, da die imprägnierende Wirkung je nach Art der Prothese während dieser Zeit aufgrund des einwachsenden Bindegewebes nicht mehr erforderlich ist.

Die Beschichtung aus resorbierbarem Material, die bei einem flächigen Implantat auf nur einer Seite oder auch auf beiden Seiten vorgesehen sein kann und gegebenenfalls je nach Anwendungszweck auch aus unterschiedlichen Materialien bestehen kann, kann ihrerseits biologische Wirkstoffe enthalten, die während der Resorptionsdauer an die Umgebung abgegeben werden. Hier kommen vorwiegend Wirkstoffe in Frage, die kein Silber sind, beispielsweise Antibiotika mit besonderem Wirkungsspektrum oder auch Wachstumsfaktoren, Wirkstoffe mit hormoneller Wirkung und dergleichen.

Eine poröse Grundstruktur wird mit besonderem Vorteil von einem textilen Material gebildet, wie dies beispielsweise bei Gefäßprothesen und Herniennetzen der Fall ist. Hier eignen sich Gewirke, Gestricke, Geflechte, Gewebe und Vliese, wobei üblicherweise Gewirke bevorzugt sind. Es können auch Kombinationen der textilen Strukturen in Frage kommen, beispielsweise Gewirke, die eine Vliesoberschicht aufweisen. Auch poröses Sintermaterial, wie expandiertes Polytetrafluorethylen kommt als polymeres Material in Frage und ist insbesondere bei Gefäßprothesen ein häufig verwendetes polymeres Material.

Die Silberschicht ist zwar bevorzugt eine geschlossene Silberschicht. Dies bedeutet aber nicht, dass die Poren bei einer porösen Gefäßstruktur durch die Silberschicht verschlossen werden. Vielmehr passt sich die Silberschicht der Oberflächenstruktur des polymeren Materials an, so dass die Poren ihre ursprüngliche Form und Größe behalten. Dies gilt für expandiertes Polytetrafluorethylen genauso wie für textiles Fasermaterial. Bei Fasermaterial ist die Faseroberfläche mit Silber beschichtet. Bei textilem Fasermaterial ist es möglich, die Fasern oder Garne mit der Silberschicht zu versehen, bevor daraus die Grundstruktur gebildet wird. Es ist jedoch ausreichend, wenn die fertige Grundstruktur an den zugängigen bzw. gewünschten Stellen mit Silber bedampft wird, da es diese Stellen sind, die der Infektionsgefahr ausgesetzt sind und mit dem umliegenden Gewebe in Verbindung kommen.

Weitere Merkmale der Erfindung ergeben sich aus der nachfolgenden Beschreibung von bevorzugten Ausführungsformen in Verbindung den Unteransprüchen. Hierbei können die einzelnen Merkmale bei einer Ausführungsform jeweils einzeln oder zu mehreren verwirklicht sein.

### Beispiel 1

Gewirkte Doppelvelourprothesen aus Polyester werden in eine rotierbare Spannvorrichtung eingespannt, so dass sie wie ein Bündel von parallelen Röhren mit Zwischenabständen nebeneinander freitragend hängen. Die Spannvorrichtung wird in eine für die Durchführung des IBAD-Verfahrens geeignete Vakuumkammer eingesetzt, wobei eine Silberbedampfung der Gefäßprothesen unter gleichzeitigem Beschuss mit Argonionen durchgeführt wird. Die Beschichtung wird so lange durchgeführt, bis an der Außenseite der Gefäßprothesen bzw. der dort liegenden Fasern eine Dicke der Silberschicht von 1300 Å erreicht ist. Wenn erwünscht, kann vorher eine Grundierung durch Bedampfung mit anderen Metallen vorgenommen werden. Silber ist auch in die Poren bzw. Zwischenräume zwischen den Fasern der Gefäßprothesen eingedrungen, so dass die Faseroberflächen auch an diesen Stellen beschichtet sind. Dort ist die Schichtdicke aufgrund der "Schattenwirkung" bei der Bedampfung jedoch geringer.

Die so beschichteten Gefäßprothesen werden aus der Spannvorrichtung entnommen und dann in üblicherweise mit resorbierbarem Material zumindest an ihrer Außenseite unter Verschluss der porösen Struktur imprägniert. Diese Imprägnierung kann in herkömmlicher Weise mit Collagen vorgenommen werden, bei dem eine Teilvernetzung mit Glutaraldehyd vorgenommen wird. Bevorzugt ist eine ebenfalls bekannte Beschichtung mit Gelatine, die mit Diisozyanat vernetzt wird. In die Beschichtungslösung können, wie erwähnt, biologisch aktive Wirkstoffe eingebracht sein, um während der späteren Resorption der Schicht die biologische Aktivität zu entwickeln.

Bestimmungen der Silbermenge auf den Gefäßprothesen (noch ohne resorbierbare Schicht) haben ergeben, dass der Silberanteil, bezogen auf das Gesamtgewicht der metallisierten Prothese im Bereich von 0,4 bis 0,8 Gew.% liegt. Der Silberanteil hängt unter anderem von der Porosität der Grundstruktur der Gefäßprothese ab. Dicht gewirkte Strukturen besitzen ein prozentuall geringeren Silberanteil als mehr poröse Strukturen. Ferner kann durch die Verfahrensführung, wie Bewegung der lmplantate während der Bedampfung, besondere Strömungsführung der Dampf- bzw. Gasströme, Einfluss auf die Durchdringung des porösen Implantats mit Silber genommen werden. Falls beispielsweise auch eine Innenbeschichtung von rohrförmigen Prothesen mit Silber erwünscht ist, können die Prothesen auch während der Beschichtung mit Silberdampf innen durchströmt werden. Ein Wenden der Prothese vor einer wiederholten Bedampfung führt ebenfalls zu einer Innenbeschichtung.

### Vergleichsversuch

Eine Gefäßprothese nach Beispiel 1, die aber noch nicht mit der resorbierbaren Imprägnierungsschicht versehen war, wurde in Phosphatpuffer (PH 7,4) bei 37°C gelagert, wobei ein täglicher Wechsel des Phosphatpuffers vorgenommen wurde und der Silbergehalt in der vorgehenden Phosphatpufferprobe bestimmt wurde. Der Versuch erstreckte sich über die Zeitdauer von 365 Tagen. Der Silbergehalt im entnommenen Phosphatpuffer lag anfänglich bei 35 Mikrogramm/l und nahm dann schnell und nach 50 Tagen (15 Mikrogramm/l) langsam ab, wobei er nach 365 Tagen noch bei ca. 5 Mikrogramm/l lag.

Unter gleichen Bedingungen wurde eine Gefäßprothese gemäß Beispiel 1 untersucht, die mit einer resorbierbaren Imprägnierungsschicht aus mit Diisocyanat vernetzter Gelatine beschichtet war. Obwohl der Gelatine kein Silber zugesetzt wurde, zeigte sich im Phosphatpuffer anfänglich ein hoher Silbergehalt im Bereich von ca. 70 bis 80 Mikrogramm/l, der zwar etwas zurück ging aber erhöht blieb, bis die resorbierbare Schicht im wesentlichen aufgelöst war. Erst nach etwa 50 Tagen war der Silbergehalt im Phosphatpuffer auf den Wert abgesunken, den die nicht mit der Imprägnierungsbeschichtung versehene Gefäßprothese nach 50 Tagen zeigte, wonach die Abgabe der Silberionen in den Phosphatpuffer im wesentlichen gleich verlief, wie bei der Gefäßprothese ohne Imprägnierungsbeschichtung.

Dieser Vergleich zeigt, dass durch die Imprägnierungsbeschichtung die Silberschicht angegriffen war und Silberionen in die Imprägnierungsbeschichtung abgegeben wurden, die dann beschleunigt und vermehrt in den Phosphatpuffer gelangten. Danach zeigte die mit der Imprägnierungsschicht versehene Gefäßprothese eine vergleichbare Abgabe von Silberionen, was bedeutet, dass die anfängliche starke Silberabgabe keinen negativen Effekt auf die Langzeitwirkung zeigt.

### Gewebereaktion

Gefäßprothesen, die in ähnlicher Weise hergestellt, aber mit Silberschichten von 1600 Å und 2500 Å versehen wurden, wurden Ratten, Kaninchen und Schweinen implantiert. Bei der Explantation nach 3 bzw. 6 Monaten zeigte sich eine gute Integration. Alle Implantate waren ohne Befund. Auch die inneren Organe waren ohne Befund. Es zeigten sich keine chronischen Entzündungsreaktionen.

### Künstliche Infektion

Zum Vergleich wurden erfindungsgemäße Implantate und solche, die anstelle einer Silberschicht auf der Grundstruktur eine Einlagerung von Silberacetat in der resorbierbaren Beschichtung enthielten, herangezogen. Die Vergleichsproben wurden künstlich mit Problemkeimen infiziert und in Kaninchen implantiert. Die Explantation erfolgte nach 7 Tagen. Danach wurden die Vergleichsproben 48 Stunden in caso-Bouillon inkubiert, wonach eine Keimzahlprüfung durchgeführt wurde. Bei 36 Proben wurde die Keimzahlbesiedelung mikrobiologisch bestimmt. Es zeigte sich, dass bei den erfindungsgemäßen Implantaten lediglich 22 %, d.h. 8 Implantate mit einer geringen Anzahl von Keimen besiedelt waren, während bei den Implantaten mit Silberacetat in der resorbierbaren Beschichtung 67 % entsprechend 23 Implantaten infiziert waren.

## Patentansprüche

1. Implantat mit antibiotischer Langzeitwirkung, insbesondere Gefäßprothese, mit einer die Form des Implantates vorgebenden Grundstruktur aus im wesentlichem nicht oder nur langsam resorbierbarem polymerem Material und einer Beschichtung aus einem resorbierbaren Material, wobei sich auf dem polymeren Material und unter der Beschichtung eine Schicht aus metallischem Silber befindet.

2. Implantat nach Anspruch 1, **dadurch gekennzeichnet, dass** die Silberschicht auf dem Polymermaterial fest haftet, insbesondere in diesem verankert ist.

3. Implantat nach Anspruch 1 oder 2, **dadurch gekennzeichnet, dass** die Silberschicht auf die Polymeroberfläche aufgedampft ist.

4. Implantat nach einem der vorhergehenden Ansprüche, **dadurch gekennzeichnet, dass** Silberatome der Silberschicht in die Polymeroberfläche der Grundstruktur eingeprägt sind, insbesondere durch Beschuss mit Argonionen in die Polymeroberfläche eingedrungen sind.

5. Implantat nach einem der vorhergehenden Ansprüche, **dadurch gekennzeichnet, dass** die Silberschicht eine im wesentlichen geschlossene Schicht ist und insbesondere so dick ist, dass sie in vivo eine Verweildauer von mehr als einem Jahr insbesondere mehr als zwei Jahren besitzt und während dieser Zeit Silberionen abgibt.

6. Implantat nach einem der vorhergehenden Ansprüche, **dadurch gekennzeichnet, dass** die Silberschicht so dick ist, dass beim Abbau im Körper maximal etwa 5 bis 10 %, insbesondere maximal 7 bis 8 %, der Schicht pro Jahr abgetragen werden.

7. Implantat nach einem der vorhergehenden Ansprüche, **dadurch gekennzeichnet, dass** die Silberschicht eine Schichtdicke von 2500 bis 1000 Å, insbesondere von ca. 1300 Å besitzt.

8. Implantat nach einem der vorhergehenden Ansprüche, **dadurch gekennzeichnet, dass** die Silberschicht ausschließlich aus elementarem Silber besteht.

9. Implantat nach einem der vorhergehenden Ansprüche, **dadurch gekennzeichnet, dass** die Grundstruktur porös ist, die Silberschicht die Poren offen lässt und die resorbierbare Schicht eine die Poren des Implantats abdichtende Imprägnierung ist.

10. Implantat nach einem der vorhergehenden Ansprüche, **dadurch gekennzeichnet, dass** die resorbierbare Schicht aus gegebenenfalls vernetztem biologischem Material gebildet ist.

11. Implantat nach einem der vorhergehenden Ansprüche, **dadurch gekennzeichnet, dass** die resorbierbare Schicht aus in vivo resorbierbarem, insbesondere abbaubaren, synthetischen Polymeren und Copolymeren, insbesondere solchen aus mindestens einer Hydroxysäure gebildet ist.

12. Implantat nach einem der vorhergehenden Ansprüche, **dadurch gekennzeichnet, dass** die Zusammensetzung der resorbierbaren Schicht so gewählt ist, dass sie spätestens nach vier Monaten, insbesondere spätestens nach ca. 40 Tagen, resorbiert ist.

13. Implantat nach einem der vorhergehenden Ansprüche, **dadurch gekennzeichnet, dass** die Beschichtung aus resorbierbarem Material ihrerseits Wirkstoffe enthält, die während der Resorption der resorbierbaren Schicht abgegeben werden.

14. Implantat nach einem der vorhergehenden Ansprüche, **dadurch gekennzeichnet, dass** die Grundstruktur von einem textilen Material, insbesondere einem Gewirk, gebildet wird.

15. Implantat nach Anspruch 14, **dadurch gekennzeichnet, dass** die Fasern der textilen Grundstruktur mindestens an den Stellen mit Silber beschichtet sind, die nach mindestens einer Oberfläche des Implantats weisen, wobei vorzugsweise im wesentlichen die gesamte Oberfläche der Fasern mit Silber beschichtet ist.

16. Implantat nach einem der Ansprüche 1 bis 13, **dadurch gekennzeichnet, dass** die Grundstruktur von einem gesinterten Material, insbesondere expandiertem Polytetrafluorethylen gebildet wird.

17. Implantat nach einem der vorhergehenden Ansprüche, **dadurch gekennzeichnet, dass** es als Prothese zum Ersatz von Hohlorganen, insbesondere als Gefäßprothese ausgebildet ist.

## Claims

1. Implant with antibiotic long-term action, in particular a vascular prosthesis, with a basic structure which defines the form of the implant and which is made of substantially non-absorbable or only slowly absorbable polymer material and of a coating of an absorbable material, with a layer of metallic silver situated on the polymer material and underneath the coating.

2. Implant according to Claim 1, **characterized in that** the silver layer adheres firmly on the polymer material, and in particular is anchored in it.

3. Implant according to Claim 1 or 2, **characterized in that** the silver layer is vapour-deposited onto the polymer surface.

4. Implant according to one of the preceding claims, **characterized in that** silver atoms of the silver layer are impressed into the polymer surface of the basic structure, and in particular are forced into the polymer surface by bombardment with argon ions.

5. Implant according to one of the preceding claims, **characterized in that** the silver layer is a substantially closed layer and in particular is of such thickness that *in vivo* it has a dwell time of more than one year, in particular of more than two years, and releases silver ions during this time.

6. Implant according to one of the preceding claims, **characterized in that** the silver layer is of such thickness that, as it breaks down in the body, a maximum of about 5 to 10%, in particular a maximum of 7 to 8%, of the layer is removed per annum.

7. Implant according to one of the preceding claims, **characterized in that** the silver layer has a layer thickness of 2500 to 1000 Å, in particular of ca. 1300 Å.

8. Implant according to one of the preceding claims, **characterized in that** the silver layer is composed exclusively of elemental silver.

9. Implant according to one of the preceding claims, **characterized in that** the basic structure is porous, the silver layer leaves the pores open, and the absorbable layer is an impregnation which seals the pores of the implant.

10. Implant according to one of the preceding claims, **characterized in that** the absorbable layer is formed from optionally crosslinked biological material.

11. Implant according to one of the preceding claims, **characterized in that** the absorbable layer is made of synthetic polymers and copolymers which are absorbable, in particular degradable, *in vivo,* in particular those comprising at least one hydroxy acid.

12. Implant according to one of the preceding claims, **characterized in that** the composition of the absorbable layer is chosen such that it is absorbed at the latest after four months, in particular at the latest after ca. 40 days.

13. Implant according to one of the preceding claims, **characterized in that** the coating of absorbable material in turn contains active substances which are released during absorption of the absorbable layer.

14. Implant according to one of the preceding claims, **characterized in that** the basic structure is made from a textile material, in particular a formed-loop knit.

15. Implant according to Claim 14, **characterized in that** the fibres of the textile basic structure are coated with silver at least at the locations which point towards at least one surface of the implant, substantially the entire surface of the fibres preferably being coated with silver.

16. Implant according to one of Claims 1 to 13, **characterized in that** the basic structure is made from a sintered material, in particular expanded polytetrafluoroethylene.

17. Implant according to one of the preceding claims, **characterized in that** said implant is designed as a prosthesis for the replacement of hollow organs, in particular as a vascular prosthesis.

## Revendications

1. Implant à action antibiotique durable, en particulier prothèse de vaisseau, comprenant une structure de base préfigurant la forme de l'implant et composée d'un matériau polymère, essentiellement non résorbable ou seulement résorbable lentement, ainsi qu'un revêtement en un matériau résorbable, une couche en argent métallique se trouvant sur le matériau polymère et sous le revêtement.

2. Implant selon la revendication 1, **caractérisé en ce que** la couche d'argent adhère solidement au matériau polymère, en étant en particulier ancrée dans celui-ci.

3. Implant selon la revendication 1 ou 2, **caractérisé en ce que** la couche d'argent est vaporisée sur la surface du polymère.

4. Implant selon l'une des revendications précédentes, **caractérisé en ce que** des atomes d'argent de la couche d'argent sont incrustés dans la surface du polymère de la structure de base, en pénétrant en particulier par bombardement d'ions argon dans la surface du polymère.

5. Implant selon l'une des revendications précédentes, **caractérisé en ce que** la couche d'argent est une couche essentiellement fermée et est en particulier d'une épaisseur telle qu'elle possède in vivo une durée de séjour de plus d'une année, en particulier de plus de deux années et **en ce qu'**elle émet pendant ce temps des ions argent.

6. Implant selon l'une des revendications précédentes, **caractérisé en ce que** la couche d'argent est d'une épaisseur telle que lors de sa dégradation dans le corps, environ 5 à 10 % au maximum, en particulier 7 à 8 % au maximum de la couche sont enlevés annuellement.

7. Implant selon l'une des revendications précédentes, **caractérisé en ce que** la couche d'argent possède une épaisseur de couche de 2500 à 1000 Å, en particulier de l'ordre de 1300 Å.

8. Implant selon l'une des revendications précédentes, **caractérisé en ce que** la couche d'argent se compose exclusivement d'argent élémentaire.

9. Implant selon l'une des revendications précédentes, **caractérisé en ce que** la structure de base est poreuse, **en ce que** la couche d'argent laisse les pores ouverts et **en ce que** la couche résorbable est une imprégnation qui assure le bouchage des pores de l'implant.

10. Implant selon l'une des revendications précédentes, **caractérisé en ce que** la couche résorbable est constituée de matériau biologique, éventuellement réticulé.

11. Implant selon l'une des revendications précédentes, **caractérisé en ce que** la couche résorbable est constituée de polymères et de copolymères synthétiques, résorbables in vivo, en particulier dégradables, tels qu'en particulier ceux issus d'au moins un acide hydroxylique.

12. Implant selon l'une des revendications précédentes, **caractérisé en ce que** la composition de la couche résorbable est choisie de telle façon qu'elle soit résorbée au plus tard après quatre mois, en particulier au plus tard après environ 40 jours.

13. Implant selon l'une des revendications précédentes, **caractérisé en ce que** le revêtement en matériau résorbable contient pour sa part des principes actifs qui sont libérés au cours de la résorption de la couche résorbable.

14. Implant selon l'une des revendications précédentes, **caractérisé en ce que** la structure de base est constituée d'un matériau textile, en particulier d'un tissu à mailles.

15. Implant selon la revendication 14, **caractérisé en ce que** les fibres de la structure textile de base sont revêtues d'argent au moins aux emplacements qui indiquent au moins une surface de l'implant, la totalité en substance de la surface des fibres étant de préférence revêtue d'argent.

16. Implant selon l'une des revendications 1 à 13, **caractérisé en ce que** la structure de base est constituée d'un matériau fritté, en particulier de polytétrafluoréthylène expansé.

17. Implant selon l'une des revendications précédentes, **caractérisé en ce qu'**il est conformé en tant que prothèse pour le remplacement d'organes creux, en particulier en tant que prothèse de vaisseau.
